Europäisches Patentamt

European Patent Office

Office européen des brevets

(19)

(11) Numéro de publication: **0 038 897**
**B1**

## FASCICULE DE BREVET EUROPEEN

(45) Date de publication du fascicule du brevet:
01.02.84

(51) Int. Cl.³. **A 61 F 1/03**

(21) Numéro de dépôt: **80420150.7**

(22) Date de dépôt: **31.12.80**

(54) **Prothèse intramédullaire non scellée pour articulation de la hanche.**

(30) Priorité: **30.04.80 FR 8010134**

(43) Date de publication de la demande:
**04.11.81 Bulletin 81/44**

(45) Mention de la délivrance du brevet:
**01.02.84 Bulletin 84/5**

(84) Etats contractants désignés:
**AT BE CH DE GB IT LI LU NL SE**

(56) Documents cités:
**EP - A - 0 000 549**
**EP - A - 0 010 527**
**DE - A - 2 808 740**
**FR - A - 2 239 979**
**FR - A - 2 366 005**
**FR - A - 2 438 470**

(73) Titulaire: **Rambert, André, Les Fontanelles 10 bis, rue du Docteur Bonhomme, F-69003 Lyon (FR)**
Titulaire: **Bousquet, Gilles, Chemin de Marandon, F-42000 Saint-Etienne (FR)**

(72) Inventeur: **Rambert, André, Les Fontanelles 10 bis, rue du Docteur Bonhomme, F-69003 Lyon (FR)**
Inventeur: **Bousquet, Gilles, Chemin de Marandon, F-42000 Saint-Etienne (FR)**

(74) Mandataire: **Maureau, Pierre, Cabinet GERMAIN & MAUREAU Le Britannia - Tour C 20, Boulevard E. Déruelle, F-69003 Lyon (FR)**

EP 0 038 897 B1

## Prothèse intramédullaire non scellée pour articulation de la hanche

La présente invention concerne une prothèse intramédullaire non scellée pour articulation de la hanche.

On utilise actuellement deux types de prothèse de hanche, à savoir: les prothèses à double cupule scellée et les prothèses intramédullaires.

Les prothèses à double cupule scellée comportent une cupule métallique scellée sur la tête fraisée du fémur et une cupule en matière plastique scellée sur le cotyle. Ce type de prothèse présente l'inconvénient d'une pose difficile puisque la tête du fémur doit être préalablement fraisée. En outre, il existe des risques de nécrose de la tête sous le ciment, nécrose entraînant des cassures rendant nécessaire une nouvelle intervention chirurgicale.

Pour obtenir de bons résultats avec des tiges fémorales non scellées, il faut que l'os spongieux se corticalise, c'est-à-dire qu'il acquiert une certaine dureté. Or, ce phénomène de corticalisation se produit d'autant mieux que l'os spongieux est soumis à des contraintes mécaniques, ce qui est rarement le cas avec les tiges utilisées habituellement.

On connait cependant (FR-A-2 295 729, EP-A-549) une prothèse intramédullaire non scellée, pour articulation de la hanche, comportant deux éléments aptes à être fixés l'un à l'autre, à savoir un élément fémoral formé essentiellement d'une tige destinée à être engagée dans le canal médullaire du fémur considéré et un élément céphalique supportant une sphère adaptable à l'extrémité de l'élément fémoral non engagé dans le fémur, cette sphère étant destinée à être engagée et à prendre appui dans la cavité cotyloïdienne de la hanche correspondante.

Dans cette prothèse, d'une part la tige porte un filetage permettant sa fixation au fémur par vissage dans le canal intramédullaire et d'autre part, les moyens d'assemblage de l'élément céphalique à l'élément fémoral comprennent d'une part, un crantage des surfaces d'appui respectives assurant leur liaison en rotation dans la position angulaire désirée et, d'autre part, une vis d'immobilisation.

On conçoit aisément qu'un réglage fin de la position angulaire de l'élément céphalique par rapport à la partie fémorale nécessite un crantage fin, ce qui est incompatible avec une bonne tenue mécanique de la liaison en rotation des deux éléments. En outre, il est évident que la mise en place de cette prothèse est longue, ce qui augmente les risques de l'intervention chirurgicale.

La présente invention vise à remédier à ces inconvénients. A cet effet, dans la prothèse qu'elle concerne, et qui est du type précité, d'une part, le filetage de la tige de l'élément fémoral est conique et, d'autre part, les moyens d'assemblage des deux éléments comprennent, dans l'élément fémoral, un alésage conique à faible pente, coaxial à la tige et débouchant dans l'extrémité de celle-ci non engagée dans le canal médullaire du fémur et, dans l'élément céphalique, en saillie sur sa face d'appui contre l'extrémité précitée de l'élément fémoral, une tige conique de profil complémentaire de l'alésage conique de l'élément fémoral, et permettant la solidarisation des deux éléments par simple coincement mutuel résultant de la faible pente des cônes, des moyens étant prévus pour repérer et conserver la position angulaire de l'élément céphalique par rapport à l'élément fémoral lors de leur assemblage.

Grâce au filetage conique de la tige de son élément fémoral, cette prothèse est d'une mise en place très rapide puisque l'élément peut être vissé en peu de tours, voire même fraction de tour.

L'extrémité de l'élément fémoral non engagée dans le canal médullaire du fémur porte une collerette coaxiale à la tige dont la face inférieure est destinée à prendre appui contre l'extrémité réséquée du fémur et dont la face supérieure est destinée à servir de face d'appui à l'élément céphalique, des moyens étant prévus pour permettre son entraînement en rotation lors de son vissage.

Par exemple, ces moyens sont constitués par des trous dans lesquels peuvent être engagés des ergots d'une clé d'entraînement.

Les moyens de repérage, de guidage et de maintien de la position angulaire de l'élément céphalique par rapport à l'élément fémoral sont constitués par au moins un ergot porté par la face d'appui de l'élément céphalique et une série de trous de profil correspondant prévus sur la face supérieure de la collerette de l'élément fémoral concentriquement à son alésage conique et dans l'un choisi desquels peut être engagé l'ergot précité, avant que la tige conique de l'élément céphalique ne soit concée dans l'alésage conique de l'élément fémoral.

De préférence, les trous prévus sur la collerette de l'élément fémoral pour le repérage et le guidage de la position angulaire de l'élément céphalique et ceux pour l'entraînement en rotation de l'élément fémoral sont confondus.

Suivant encore une autre caractéristique de l'invention, les filets du filetage conique de la tige de l'élément fémoral sont revêtus de céramique d'alumine favorable à la corticalisation de l'os spongieux tel que l'oxyde d'aluminium.

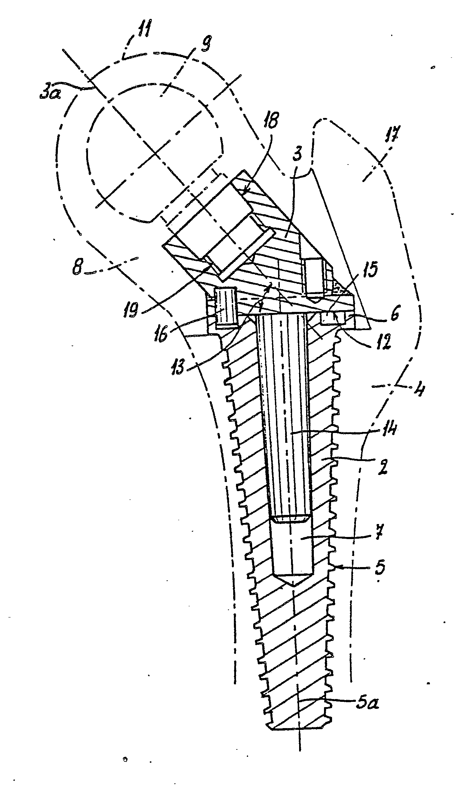

De toute façon, l'invention sera bien comprise à l'aide de la description qui suit, en référence au dessin schématique annexé dont l'unique figure représente, vue en coupe axiale, une forme d'exécution de cette prothèse donnée à titre d'exemple non limitatif.

Cette prothèse comprend essentiellement deux éléments: un élément fémoral 2 et un élément céphalique 3.

L'élément fémoral 2 se compose essentiellement d'une tige destinée à être engagée et fixée dans le canal médullaire du fémur 4 d'un patient. Comme le montre le dessin, cette tige porte extérieurement un filetage conique 5 et son extrémité de grand diamètre, c'est-à-dire celle opposée à celle qui est destinée à être engagée dans le canal médullaire du fémur 4, présente une collerette 6 coaxiale à l'axe 5a du filetage 5.

En outre, débouchant au centre de la collerette 6 cet élément fémoral 2 présente un alésage conique 7 à faible pente.

Le second élément, à savoir l'élément céphalique 3 destiné à remplacer d'une part, le col 8 du fémur 4 et, d'autre part, par une sphère 9 qu'il supporte, la tête 11 du fémur 4 est destiné à être assemblé à l'élément fémoral 2. L'élément céphalique 3 se présente sensiblement comme une pièce cylindrique. L'une 12 des extrémités de cet élément 3 qui est destinée à prendre appui sur la collerette 6 de l'élément fémoral 2 est constituée par une surface plane formant avec l'axe 3a un angle 13 correspondant à l'angle formé normalement entre l'axe du fémur 4 et l'axe de son col. Au centre de cette surface d'appui 6, fait saillie une tige conique 14 de profil complémentaire à l'alésage conique 7 de l'élément fémoral 2 et destinée à être engagée dans cet alésage pour assurer, par coincement, la fixation de l'élément céphalique 3 à l'élément fémoral 2.

Pour permettre le repérage, le guidage et le maintien de la position angulaire de l'élément céphalique 3 par rapport à l'autre autour de l'axe 5a, d'une part, la collerette 6 présente, sur sa face d'assemblage, des trous 15 répartis régulièrement suivant une couronne concentrique à l'axe 5a et, d'autre part, l'élément 3 présente en saillie sur sa face d'appui 12 un ergot 16 de même section que les trous 15 et apte à être engagé dans l'un choisi des dits trous. Cet engagement a donc pour effet d'assurer l'immobilisation en rotation des éléments 2 et 3 de cette prothèse même en présence d'efforts importants transmis par la cavité cotyloïdienne de la hanche du patient à la sphère 9 portée par l'élément céphalique 3.

Lors de la mise en place de cette prothèse, le praticien peut donc déterminer la position angulaire de l'élément céphalique 3 par rapport à l'élément fémoral 2 en fonction de l'antéversion désirée.

Pour cette mise en place, il suffit donc de fixer l'élément fémoral 2 seul dans le canal médullaire du fémur 4 en utilisant, par exemple, les trous 15 pour son entraînement en rotation. Pour cela, il suffit de disposer d'une clé à ergot engageable dans au moins deux trous 15. Il faut noter que la conicité du filetage 5 permet un engagement direct de l'élément fémoral 2 dans le canal médullaire du fémur 4 et que l'opération de vissage n'est nécessaire qu'en fin de cet engagement, ce qui permet une mise en place très rapide.

En outre, le fait de pouvoir mettre en place l'élément fémoral 2 avant l'assemblage de l'élément céphalique 3 permet de conserver au patient le grand trauchantère 17.

Comme le montre le dessin, le filetage conique 5 est à un pas relativement important, tel que de l'ordre de 4 à 6 mm, de manière à permettre sa bonne pénétration et un bon accrochage dans l'os spongieux du canal médullaire du fémur 4. En outre, ils sont avantageusement revêtus d'une céramique d'oxyde d'aluminium favorable à la corticalisation de cet os spongieux.

Comme le montre également le dessin, la sphère 9 destinée à remplacer la tête du fémur du patient peut être assemblée de manière amovible à l'élément céphalique 3 afin de faciliter les conditions de stockage de cette prothèse. Dans ce cas, cet assemblage est avantageusement réalisé comme décrit dans le brevet FR-A-2 349 319 en prévoyant d'une part, un emmanchemant conique comme montré en 18 et, d'autre part, un assemblage par vissage comme montré en 19.

Comme il va de soi et comme il ressort de ce qui précède, l'invention ne se limite pas à la seule forme d'exécution de cette prothèse qui a été décrite ci-dessus à titre d'exemple non limitatif; elle en embrasse, au contraire, toutes les variantes de réalisation.

**Revendications**

1. Prothèse intramédullaire non scellée pour l'articulation de la hanche du type comportant deux éléments aptes à être fixés l'un à l'autre, à savoir, un élément fémoral (2) formé essentiellement d'une tige filetée destinée à être engagée dans le canal médullaire du fémur (4) considéré et un élément céphalique (3) supportant une sphère (9) adaptable à l'extrémité de la partie fémorale (2) non engagée dans le fémur (4), cette sphère (9) étant destinée à être engagée et à prendre eppui dans la cavité cotyloïdienne de la hanche correspondante, caractérisée en ce que, d'une part, le filetage (5) de la tige de l'élément fémoral (2) est conique et, d'autre part, les moyens d'assemblage des deux éléments fémoral (2) et céphalique (3) comprennent, dans l'élément fémoral (2), un alésage conique (7) à faible pente, coaxial à la tige et débouchant dans l'extrémité de celle-ci non engagée dans le canal médullaire du fémur (4) et, dans l'élément céphalique (3), en saillie sur sa face d'appui (12) contre l'extrémité précitée de l'élément fémoral (2), une tige conique (14) de profil complémentaire de l'alésage conique (7) de l'élément fémoral (2), et permettant la solidarisation des deux éléments par simple coincement mutuel résultant de la faible pente des cônes, en ce que l'extrémité de l'élément fémoral (2) non engagée dans le canal médullaire du fémur (4) porte une collerette (6) coaxiale à la tige, dont la face inférieure est destinée à prendre appui contre l'extrémité réséquée du fémur (4), dont la face supérieure est destinée à servir de face d'appui à l'élément céphalique (3) et qui est munie de moyens pour permettre son entraînement en rotation lors de son vissage, et en ce que sont prévus au moins un ergot (16) porté par la face d'appui (12) de l'élément céphalique (3), et une série de trous (15) de profil correspondant ménagés sur la face supérieure de la collerette (6) de l'élément fémoral (2) concentriquement, à son alésage conique (7) et dans l'un choisi desquels peut être engagé l'ergot (16) précité, avant que la tige conique de l'élément céphalique (3) ne soit coincée dans l'alésage conique (7) de l'élément fémoral (2).

2. Prothèse selon la revendication 1, caractérisée en ce que les moyens d'entraînement en rotation de l'élément fémoral (2) en vue de son vissage dans le canal médullaire du fémur (4) sont constitués par des trous (15) ménagés dans sa face supérieure, concentriquement à son alésage conique (7) et dans lesquels peuvent être engagés les ergots d'une clé d'entraînement.

3. Prothèse selon la revendication 2, caractérisée en ce que les trous (15) prévus sur la collerette (6) de l'élément fémoral (2) pour le repérage, le guidage et le maintien de la position angulaire de l'élément céphalique (3) et ceux pour l'entraînement en rotation de l'élément fémoral (2) sont confondus.

4. Prothèse selon l'une quelconque des revendications précédentes, caractérisée en ce qu la sphère (9) destinée à remplacer la tête (11) du fémur (4) du patient est fixée de manière amovible à l'élément céphalique (3).

5. Prothèse selon l'une quelconque des revendications précédentes, caractérisée en ce que les filets du filetage conique (5) de la tige de l'élément fémoral (2) sont revêtus de céramique d'aluminium favorable à la corticalisation de l'os spongieux, tel que l'oxyde d'aluminium.

### Patentansprüche

1. Nicht zementierte Endoprothese für ein Hüftgelenk, des Typs mit zwei Elementen, die aneinander befestigbar sind, nämlich ein Femoralelement (2), das im wesentlichen aus einer mit Gewinde versehenen, in den Markkanal des betroffenen Oberschenkels (4) einbringbaren Gewindestange gebildet ist, und einem Kopfelement (3), das eine an das nicht in den Oberschenkel (4) eingebrachte Ende des Femoralelementes (2) anpaßbare Kugel (9) trägt, welche Kugel (9) dazu bestimmt ist, in die pfannenförmige Höhlung der entsprechenden Hüfte eingebracht und dann abgestützt zu werden, dadurch gekennzeichnet, daß einerseits das Gewinde (5) der Stange des Femoralelementes (2) konisch ist und andererseits die Verbindungsmittel des Femoralelementes (2) und des Kopfelementes (3) im Femoralelement (2) eine konische Bohrung (7) mit schwacher Neigung, coaxial zur Stange und in ihrem nicht in den Markkanal des Oberschenkels (4) eingesteckten Ende mündend, und im Kopfelement (3), vorspringend von seiner Abstützfläche (2) gegenüber dem vorgenannten Ende des Femoralelementes (2), eine konische Stange (14) beinhalten, deren Profil komplementär zur konischen Bohrung (7) des Femoralelementes (2) ist und die die feste Verbindung der beiden Elemente durch einfaches gegenseitiges Verkeilen, resultierend aus der schwachen Neigung der Konen, ermöglicht, und daß das Ende des nicht in den Markkanal des Oberschenkels (4) eingesteckte Ende des Femoralelementes (2) einen zu der Stange coaxialen Bund (6) trägt, dessen untere Fläche dazu bestimmt ist, sich gegen das resezierte Ende des Oberschenkels (4) abzustützen und dessen obere Fläche dazu bestimmt ist, als Abstützfläche des Kopfelementes (3) zu dienen und der mit Mitteln versehen ist, die seinen Drehantrieb bei der Verschraubung ermöglichen, und daß ferner mindestens ein Zapfen (16), getragen von der Abstützfläche (12) des Kopfelementes (3) sowie eine Reihe von Löchern (5) entsprechenden Profiles vorgesehen sind, die in der oberen Fläche des Bundes (6) des Femoralelementes (2) konzentrisch zu der Bohrung (7) vorgesehen sind, wobei in ein ausgewähltes von ihnen der Zapfen (16) eingesteckt werden kann, bevor die konische Stange des Kopfelementes (3) in der konischen Bohrung (7) des Femoralelementes (2) verkeilt ist.

2. Prothese nach Anspruch 1, dadurch gekennzeichnet, daß die Drehantriebsmittel des Femoralelementes (2) im Hinblick auf seine Einschraubung in den Markkanal des Oberschenkels (4) aus Löchern (15) gebildet sind, die in seiner oberen Fläche, konzentrisch zu seiner konischen Bohrung (7) angeordnet sind und in die die Zapfen eines Antriebsschlüssels einsteckbar sind.

3. Prothese nach Anspruch 2, dadurch gekennzeichnet, daß die auf dem Bund (6) des Femoralelementes (2) für die Festlegung, die Führung und die Aufrechterhaltung der Winkellage des Kopfelementes (3) vorgesehenen Löcher (15) und diejenigen für den Drehantrieb des Femoralelementes (2) zusammenfallen.

4. Prothese nach einem der vorhergehenden Ansprüche, dadurch gekennzeichnet, daß die den Kopf (11) des Oberschenkels (4) des Patienten ersetzende Kugel (9) lösbar an dem Kopfelement (3) befestigt ist.

5. Prothese nach einem der vorhergehenden Ansprüche, dadurch gekennzeichnet, daß die Gewindegänge des konischen Gewindes (5) der Stange des Femoralelementes (2) mit einem keramischen Material auf Aluminiumbasis, beispielsweise Aluminiumoxyd, das die Corticalisation des Schwammanteiles des Knochens begünstigt, überzogen sind.

### Claims

1. An unsealed intramedullary prothesis for articulation of the hip, of the type comprising two units suitable to be fitted to one another, namely a femoral unit (2) essentially composed of a threaded stem intended to be engaged into the medullary channel of the femur in question, and a cephalic unit (3) supporting a sphere (9), capable of being fitted to the end of the femoral portion (2) that is not engaged into the femur (4), this sphere (9) being intended to be engaged into and to rest against the cotyloid cavity of the corresponding hip, characterized in that on one hand the screw thread (5) on the stem of the femoral unit (2) is conical and, on the other hand the means of assembly of the two femoral (2) and cephalic (3) units comprise, in the femoral unit (2) a conically reamed hole of small taper, coaxial with the stem and opening at the end of the latter that is not fitted into the medullary channel of the femur (4), and in the cephalic unit (3) a conical stem (14) of profile complementary to that of the conically reamed hole (7) of the femoral unit (2), projecting from the surface (12) of the cephalic unit (3) that fits against the above-mentioned free end of the femoral unit (2) and enabling the two units to be solidly held together by a simple mutual wedging action resulting from the small taper of the cones; in that the end of the femoral unit (2) that is not engaged into the medullary channel of the femur (4) carries a small collar (6) coaxial with the stem, the lower face of which collar is intended to bear against the resected end of the femur (4) and the upper face of which collar is intended to act as support face for the cephalic unit (3) and is provided with means for it to be rotated as it is screwed up; and in that at least one peg (16) is provided, carried on the contact face (12) of the cephalic unit (3), and a series of holes (15) of corresponding profile is provided on the upper face of the small collar (6) of the femoral unit (2) concentric with its conically reamed hole (7), into a selected hole of which series the series the peg (16) may be inserted before the conical stem of the cephalic unit (3) is wedget into the conically reamed hole (7) of the femoral unit (2).

2. A prothesis according to Claim 1, characterized in that the means of rotating the femoral unit (2) for the purpose of screwing it into the medullary channel of the femur (4) consist of holes (15) provided on its upper surface concentric with its conically reamed hole (7), into which holes the pegs of a driving key may be inserted.

3. A prothesis according to Claim 2, characterized in that the holes (15) provided on the small collar (6) of the femoral unit (2) for location, guidance and retention of the angular position of the cephalic unit (3), and those for rotating the femoral unit (2), are identical.

4. A prothesis according to any of the preceding Claims, characterized in that the sphere (9) intended to replace the head (11) of the patient's femur (4) is fixed to the cephalic unit (3) in a rigid manner.

5. A prothesis according to any of the preceding Claims, characterized in that the threads of the conical screw (5) of the stem of the femoral unit (2) are coated with aluminium ceramic material, such as aluminium oxide, that promotes the corticalization of spongy bone.